# EUROPÄISCHE PATENTSCHRIFT

(11) **EP 0 939 319 B1**
(45) Veröffentlichungstag und Bekanntmachung des Hinweises auf die Patenterteilung: **30.11.2005**
(21) Anmeldenummer: 99103135.2
(22) Anmeldetag: 17.02.1999
(51) Int. Cl.: G01N 33/543

(54) **Erzeugung räumlich scharf begrenzter Festphasen für Bindungsassays**
Production of spatially defined solid phases for binding assays
Production de phases solides spatialement définies pour l'essai à liaison

(30) Priorität: 19.02.1998 DE 19806989
(43) Veröffentlichungstag der Anmeldung: 01.09.1999
(60) Teilanmeldung: 03019025.0
(73) Patentinhaber: Roche Diagnostics GmbH, 68305 Mannheim-Waldhof (DE)
(72) Erfinder: Hornauer, Hans Dr., 82380 Peissenberg (DE)
(74) Vertreter: Weiss, Wolfgang

(56) Entgegenhaltungen:
- EP-A- 0 427 984
- EP-A- 0 529 775
- US-A- 4 591 570
- AKMAN SERIF ET AL: "An enzymeimmunoassay for total thyroxine using avidin-biotin separation system and thyroxine-peroxidase conjugate." JOURNAL OF IMMUNOASSAY, Bd. 16, Nr. 3, 1995, Seiten 325-341, XP000945253 ISSN: 0197-1522
- PIRRUNG MICHAEL C ET AL: "A general method for the spatially defined immobilization of biomolecules on glass surfaces using "caged" biotin." BIOCONJUGATE CHEMISTRY, Bd. 7, Nr. 3, 1996, Seiten 317-321, XP002095758 ISSN: 1043-1802
- HETLAND O ET AL: "Cardiac troponin T immunoassay on biotin-streptavidin-coated microplates: Preliminary performance in acute myocardial infarction." SCANDINAVIAN JOURNAL OF CLINICAL AND LABORATORY INVESTIGATION, Bd. 55, Nr. 8, 1995, Seiten 701-713, XP000945237 ISSN: 0036-5513
- Methods of Immunological Analysis, edited by Masseyeff R.F. et al, VCH Verlag, Bd. 1, Fundamentals, 1993, Seiten 507 bis 517

## Beschreibung

Die Erfindung betrifft ein Verfahren zum Aufbringen einer mehrlagigen Beschichtung auf einen festen, nicht porösen Träger sowie Festphasen, die mit dem erfindungsgemäßen Verfahren hergestellt werden.

Bindungsassays zur Bestimmung von Analyten, wie etwa Immunoassays, Nukleinsäurehybridisierungsassays usw. werden in großem Umfang eingesetzt. Der Nachweis eines Analyten durch Bindungsassays kann mit einer heterogenen Testführung erfolgen, wobei eine flüssige Phase mit einer Festphase in Kontakt gebracht wird. Die Festphase umfaßt Testflächen mit Rezeptormolekülen, an die der Analyt spezifisch bindefähig ist. Die Bindung des Analyten wird anschließend, z.B. mittels einer Markierung, nachgewiesen.

Für eine zuverlässige qualitative oder quantitative Bestimmung eines Analyten, ist es notwendig, die Testflächen der Bindungsassays reproduzierbar und mit genau definierten Mengen an Rezeptormolekülen herstellen zu können. Unterschiede in der Größe der Testflächen, Randunschärfe der Testflächen, Verschmieren der Rezeptormoleküle, unterschiedliche Rezeptordichte in den Testflächen und unspezifische Bindestellen führen zu Unterschieden in der detektierten Signalhöhe und somit zu Ungenauigkeiten bei der Auswertung von Testergebnissen.

Diese Probleme wirken sich zumeist umso stärker aus, je kleiner die Festphase und die darauf befindlichen Testflächen sind. Liegen einzelne Testflächen mit unterschiedlichen Rezeptormolekülen zum Nachweis verschiedener Analyten nahe beieinander, so kann es durch Randunschärfe der Testflächen oder/und Verschmieren der Rezeptormoleküle zur Kontamination spezifischer Testflächen mit gegen einen anderen Analyten gerichteten Rezeptormolekülen kommen, was zu falschen Testergebnissen führen kann. Besonders schwerwiegend ist dieses Problem bei qualitativen Tests zum Nachweis von Infektionskrankheiten. So hat z.B. eine aufgrund von Verschmieren der Rezeptormoleküle falsch positive Probe bei einem HIV-Test erhebliche Konsequenzen.

Eine Vielzahl von Testformaten beruht auf der Verwendung von porösen Trägermaterialien wie etwa Papierscheiben oder anderen porösen Materialien. Bei solchen Testformaten, wie sie beispielsweise in der EP-A-0 427 984 und der EP-A-0 063 810 beschrieben sind, wird eine Rezeptorschicht auf einem porösen Material gebildet, indem Rezeptormoleküle aus einer Lösung auf das poröse Material aufgebracht und durch das Trägermaterial aufgesaugt wurden. Das Aufsaugen der Beschichtungslösung bewirkt insbesondere eine von der lokalen Mikrostruktur des Trägermaterials abhängige Aufweitung der beschichteten Fläche. Es ist deshalb schwierig, mit dieser Methode reproduzierbare gleichmäßige Testflächen, insbesondere in miniaturisierten Testformaten bereitzustellen. Zudem ist bei solchen miniaturisierten Testformaten auf Basis von porösen Trägermaterialien die Auswertung schwierig. Durch die Unebenheit und Ungleichmäßigkeit der Oberfläche poröser Materialien kann zumeist nur eine begrenzte optische Auflösung der Testflächen erreicht werden.

Es wurde deshalb versucht, anstelle von porösem Trägermaterial ein Trägermaterial mit einer nichtporösen Oberfläche zu verwenden. US-PS-4,591,570 beschreibt eine Immunoassay-Vorrichtung zur gleichzeitigen Bestimmung mehrerer Antigene mit Hilfe eines Antikörperarrays, wobei als Träger Glas oder Kunststoff verwendet wird. Durch die Verwendung dieses Trägermaterials ist es möglich, die Gesamtgröße des Testformats und somit die benötigte Probenmenge deutlich zu verringern. Allerdings tritt auch bei nichtporösen Oberflächen, insbesondere bei miniaturisierten Testverfahren, das Problem auf, daß die Rezeptormoleküle verschmieren. Ein Verschmieren kann durch unkontrolliertes Spriten der aufgebrachten Beschichtungslösung, d.h. eine Flächenzunahme, bei der die Kontur regelmäßig bleibt, verursacht werden. Daneben kann auch ein unkontrolliertes Zerfließen der aufgebrachten Beschichtungslösung auftreten, was zu einer unregelmäßigen Kontur führt. Schließlich kann das Verschmieren eine Verfrachtung von Rezeptormolekülen in den Bereich außerhalb der mit Beschichtungslösung benetzten Flächen bewirken.

Viele der üblicherweise verwendeten Rezeptormoleküle, wie etwa niedermolekulare Rezeptormoleküle, adsorbieren zudem nur schlecht oder schlecht reproduzierbar auf festen Trägern mit nichtporösen Oberflächen. Weiterhin enthalten viele wässrige Lösungen von Rezeptormolekülen, wie sie üblicherweise zum Aufbringen von Rezeptormolekülen auf Festphasen verwendet werden, zum Stabilisieren ein Detergens, wodurch die Adsorption der Rezeptormoleküle auf feste Träger mit nichtporösen Oberflächen zumindest zum Teil verhindert wird.

Um die Bindefähigkeit von Rezeptormolekülen an nichtporöse Oberflächen zu verbessern, können mehrlagige Beschichtungen aufgebracht werden. Aber auch bei solchen Beschichtungen treten innerhalb einzelner Testflächen Unterschiede in der Größe und Randunschärfen auf, was bei der Auswertung, insbesondere von miniaturisierten Testformaten, erhebliche Probleme ergibt.

Eine Aufgabe der vorliegenden Erfindung war es deshalb, ein Verfahren bereitzustellen, mit dem räumlich scharf begrenzte Testflächen für Bindungsassays erhalten werden können.

Diese Aufgabe wird erfindungsgemäß gelöst durch ein Verfahren zum Aufbringen einer mehrlagigen Beschichtung auf einen festen, nichtporösen Träger, umfassend die Schritte:
(a) Aufbringen einer Vorbeschichtung auf ein Reagenzfeld des festen Trägers,
(b) Waschen des vorbeschichteten Trägers mit einer wässrigen Flüssigkeit, die eine Konzentration an Puffersubstanzen von ≤ 250 mmol aufweist, und
(c) Aufbringen einer zweiten Beschichtung in Form von Mikrotröpfchen, umfassend Rezeptormoleküle, die mit der Vorbeschichtung-bindefähig sind, auf den vorbeschichteten Träger in Form von räumlich begrenzten Flächen auf dem Reagenzfeld.

Erfindungsgemäß wird der feste, nichtporöse Träger zunächst mit einer Vor-beschichtung versehen. Die Vorbeschichtung kann dabei vollflächig auf einen Teil oder auf die gesamte Fläche eines Reagenzfeldes des festen Trägers oder auch in Form von Spots aufgetragen werden. Bevorzugt wird die Vorbeschichtung vollflächig aufgetragen. Die Vorbeschichtung wird typischerweise aus einer wässrigen Lösung auf den Träger aufgebracht. Sie kann aus beliebigen Molekülen bestehen, welche die Bindung einer zweiten Beschichtung ermöglichen. Bevorzugt umfaßt die Vorbeschichtung einen ersten Partner eines hochaffinen Bindepaares, wie z.B. Streptavidin, Avidin oder Biotin sowie Analoga, Derivate und Konjugate der vorstehenden Substanzen, oder Antikörper wie z.B. Anti-Maus-Antikörper. Es können aber auch Moleküle als Vorbeschichtung aufgebracht werden, die für eine kovalente Bindung mit der zweiten Beschichtung vorgesehen sind, etwa Moleküle, die eine Amin-, eine Sulfid- oder eine Silylgruppe enthalten.

Wenn man auf eine solche Vorbeschichtung Rezeptormoleküle in wässriger Lösung in Form kleiner Tröpfchen aufbringt, diffundieren sie aus der Lösung an die Vorbeschichtung und binden an diese. Es wurde jedoch festgestellt, daß die aufgebrachten Flüssigkeitströpfchen verlaufen, was zu unterschiedlicher Kontur und Größe der Spotflächen führt. Daraus resultieren Unterschiede in der Rezeptordichte in den einzelnen Testflächen und somit Unterschiede in der Signalhöhe die zu falschen Resultaten führen können.

Überraschenderweise wurde nun festgestellt, daß durch Einführen des Verfahrenschrittes (b), nämlich Waschen des vorbeschichteten Trägers mit einer wässrigen Flüssigkeit, reproduzierbare, gleichförmige Testspots erhalten werden können. Das Waschen des vorbeschichteten Trägers kann mit reinem Wasser durchgeführt werden, bevorzugt wird ein Puffer mit geringer lonenstärke verwendet. Die wässrige Flüssigkeit oder Waschlösung, die zum Waschen des vorbeschichteten Trägers verwendet wird, kann Puffersubstanzen, Detergenzien oder/und sonstige Zusätze umfassen. Als Puffersubstanzen können grundsätzlich alle gebräuchlichen oder dem Fachmann bekannten Puffersubstanzen verwendet werden. Die Konzentration, in der die Puffersubstanzen in der Waschlösung vorliegen beträgt ≤ 250 mM, bevorzugt ≤ 100 mM und am meisten bevorzugt ≤ 40 mM. Die besten Ergebnisse im Hinblick auf die erfindungsgemäße Funktion als Waschlösung werden dann erhalten, wenn die Puffersubstanz in einer Konzentration ≤ 10 mM, bevorzugt ≤ 5 mM und am meisten bevorzugt ≤ 2 mM vorliegt. In Tabelle 1 sind einige Puffersubstanzen, sowie ihre bevorzugten (c₁), besonders bevorzugten (c₂) und am meisten bevorzugten (c₃) Konzentrationsbereiche angegeben.

**Tabelle 1**

| **Puffersubstanz** | **c**_{**1**} **[mM]** | **c**_{**2**} **[mM]** | **c**_{**3**} **[mM]** |
|---|---|---|---|
| Phosphat | ≤ 40 | ≤ 5 | ≤ 2 |
| Carbonat | ≤ 40 | ≤ 5 | ≤ 2 |
| Mes/Tris | ≤ 100 | ≤ 10 | ≤ 5 |
| Hepes/Tris | ≤ 100 | ≤ 10 | ≤ 5 |

Für eine weitere Optimierung des erfindungsgemäßen Verfahrens ist es möglich, in Abhängigkeit von der Art der Wechselwirkung, mit der die Rezeptormoleküle auf der Vorbeschichtung gebunden werden, einen Puffer auszuwählen, der optimale Reaktionsbedingungen gewährleistet. Beispielsweise ist für eine Vorbeschichtung mit Streptavidin der pH-Bereich von 5 bis 9 optimal.

Erfindungsgemäß können auch Pufferkombinationen verwendet werden, die eine flüchtige Komponente enthalten, wie etwa Essigsäure oder Ameisensäure. Beim Eintrocknen von Pufferresten können solche Pufferkombinationen jedoch auf der Vorbeschichtung zu großen pH-Schwankungen führen, da lediglich ein Bestandteil des Puffers verdampft, während der andere auf der Oberfläche verbleibt. Bevorzugt werden deshalb Pufferkombinationen verwendet, die ausschließlich aus flüchtigen oder ausschließlich aus nichtflüchtigen Komponenten bestehen.

Zusätzlich oder anstelle von Puffersubstanzen kann die erfindungsgemäß verwendete Waschlösung auch ein Detergenz enthalten. Ein Zusatz von Detergenz zur Waschlösung unterstützt das weitgehend rückstandsfreie Entfernen der Waschlösung von der Vorbeschichtung. Das Detergenz ist in der Waschlösung bevorzugt in einer Konzentration ≤ 0,05 Vol.-% (bezogen auf das Gesamtvolumen), insbesondere ≤ 0,02 % (vol-vol), mehr bevorzugt ≤ 0,01 % (vol-vol) und am meisten bevorzugt ≤ 0,005 % (vol-vol) enthalten. Die besten Ergebnisse werden erhalten, wenn die Konzentration des Detergenz in der Waschlösung ≤ 0,003 % (vol-vol), mehr bevorzugt ≤ 0,002 % (vol-vol) und am meisten bevorzugt ≤ 0,001 % (vol-vol) beträgt. Die bevorzugten (c₁), besonders bevorzugten (c₂) und am meisten bevorzugten (c₃) Konzentrationen für einige bevorzugte Detergenzien sind in Tabelle 2 angegeben.

**Tabelle 2**

| **Detergenz** | **c**_{**1**} **[% vol-vol)]** | **c**_{**2**} **[% (vol-vol)]** | **c**_{**3**} **[% (vol-vol)]** |
|---|---|---|---|
| Tween 20 | ≤ 0,01 | ≤ 0,005 | ≤ 0,003 |
| n-Octylglucosid | ≤ 0,02 | ≤ 0,01 | ≤ 0,003 |
| Chaps | ≤ 0,01 | ≤ 0,005 | ≤ 0,003 |
| Brj 35 | ≤ 0,005 | ≤ 0,002 | ≤ 0,001 |

Grundsätzlich sind alle Detergenzien geeignet, ein weitgehend rückstandsfreies Entfernen der Waschlösung zu erzielen. Die Eignung eines bestimmten Detergenz für ein bestimmtes Testformat kann abhängig vom Typ der Beschichtung und dertechnischen Randbedingungen, wie etwa dem Format des Reagenzträgers, der verwendeten Absaugvorrichtung usw. vom Fachmann durch wenige Vorversuche ermittelt werden. Insbesondere muß darauf geachtet werden, daß das Detergenz eine später aufgebrachte Flüssigkeit nicht oder nur geringfügig zum Zerfließen bringt. Aus diesem Grund ist beispielsweise bei der Verwendung des Detergenz SDS besondere Aufmerksamkeit, insbesondere hinsichtlich der verwendeten Konzentration angebracht.

Die erfindungsgemäß verwendete Waschlösung kann auch sonstige Zusätze umfassen, die sich vorteilhaft auf die Verfahrensführung auswirken. Insbesondere können Zusätze verwendet werden, die stabilisierend auf die Vorbeschichtung wirken. Dies ermöglicht eine zeitliche Trennung zwischen der Vorbeschichtung des Reagenzträgers und der Aufbringung der für den jeweiligen Bindeassay spezifischen Beschichtung im Herstellungsverfahren. Beispiele für solche sonstige Zusätze sind Zucker, Salze und Blockreagenzien. Die bevorzugten (c₁), besonders bevorzugten (c₂) und am meisten bevorzugten (c₃) Konzentrationen für diese Substanzen sind in Tabelle 3 dargestellt.

**Tabelle 3**

| **Substanz** | **c**_{**1**} **[% (Gew.-Vol)]** | **c**_{**2**} **[% (Gew.-Vol)]** | **c**_{**3**} **1% (Gew.-Vol)]** |
|---|---|---|---|
| Zucker | ≤ 0,5 | ≤ 0,1 | ≤ 0,05 |
| Salze | ≤ 0,5 | ≤ 0,1 | ≤ 0,05 |
| Blockreagenzien | ≤ 0,2 | ≤ 0,02 | ≤ 0,005 |

Bevorzugte Zucker sind niedermolekulare Zucker, wie etwa Saccharose, mit einem Molekulargewicht ≤ 1000, besonders bevorzugt ≤ 500. Am meisten bevorzugt sind niedermolekulare Zuckeroligomere, die eine, zwei oder drei Zuckereinheiten umfassen.

Als Salze werden bevorzugt solche verwendet, die während des Eintrocknens nicht zur Kristallbildung neigen, wie etwa Di-Natriumtatrat-dihydrat.

Als Blockreagenzien werden bevorzugt niedermolekulare Proteine, wie etwa Pepton verwendet.

Die Konzentration der einzelnen Stoffe in der Waschlösung kann in Abhängigkeit von den spezifischen Testanforderungen, wie etwa Art der Beschichtung, Größe des Testformats usw. eingestellt werden, so wird beispielsweise im Fall von Reagenzträgern mit relativ großen Reaktionsflächen (Druchmesser > 1 cm), bevorzugt reines Wasser oder eine Waschlösung mit relativ geringen Konzentrationen der oben genannten Substanzen (Puffer, Detergenz, sonstige Zusätze) verwendet, um eine möglichst rückstandsfreie Absaugung zu erzielen.

Bevorzugt wird die wässrige Flüssigkeit über die Vorbeschichtung geleitet und dann vollständig entfernt, z.B. abgesaugt, so daß die Vorbeschichtung trocken zurückbleibt. Bevorzugt wird eine Vorbeschichtung verwendet, die hydrophobe Eigenschaften aufweist.

Auf die gewaschene Vorbeschichtung wird dann eine zweite Beschichtung aufgebracht, umfassend Rezeptormoleküle, die mit der Vorbeschichtung bindefähig sind, und zwar in Form von räumlich begrenzten Flächen auf dem Reagenzfeld. Die Rezeptormoleküle enthalten bevorzugt den zweiten Partner des Bindepaares, der eine hochaffine Wechselwirkung, z.B. eine immunologische Reaktion, eine Streptavidin/Avidin-Wechselwirkung oder ähnliches oder auch eine kovalente Bindung mit dem als Vorbeschichtung aufgebrachten ersten Partner des Bindepaares eingehen kann. So kann beispielsweise als Vorbeschichtung Streptravidin oder Avidin aufgebracht werden und das Rezeptormolekül enthält dann einen Biotinanteil. Bevorzugt erfolgt die Bindung der Rezeptormoleküle an die Vorbeschichtung mittels hochaffiner Wechselwirkungen mit einer Gleichgewichtskonstante K_{M} ≥ 10⁸ 1/mol. Durch das Aufbringen der zweiten Beschichtung auf die gewaschene Vorbeschichtung erhält man reproduzierbare, gleichförmige Konturen und Größen der Testflächen.

Dieser Effekt wird durch die beigefügten Figuren weiter erläutert, worin
- Figur 1: miniaturisierte Testflächen zeigt, die auf herkömmliche Weise ohne Waschschritt hergestellt wurden,
- Figur 2: miniaturisierte Testflächen zeigt, die gemäß dem erfindungsgemäßen Verfahren hergestellt worden sind und
- Figur 3: die Wirkung des Waschschritts veranschaulicht, wobei Figur 3a ein mit Wasser gefülltes unbeschichtetes Enzymun-Teströhrchen (das ist ein unbeschichtetes Polystyrolröhrchen) zeigt, Figur 3b ein mit Wasser gefülltes Streptavidin beschichtetes Enzymun-Teströhrchen (das ist ein Streptavidin-beschichtetes Polystyrolröhrchen, Boehringer Mannheim, Best.-Nr. 1144553, "Enzymun-Test Streptavidin Tube") zeigt und Figur 3c ein Streptavidin beschichtetes und anschließend gewaschenes und getrocknetes Enzymun-Teströhrchen, in das Wasser gefüllt wurde, zeigt.

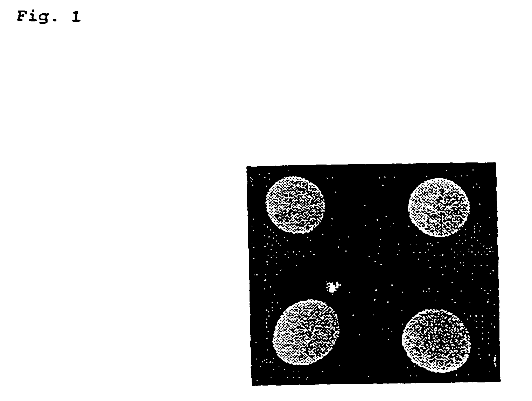

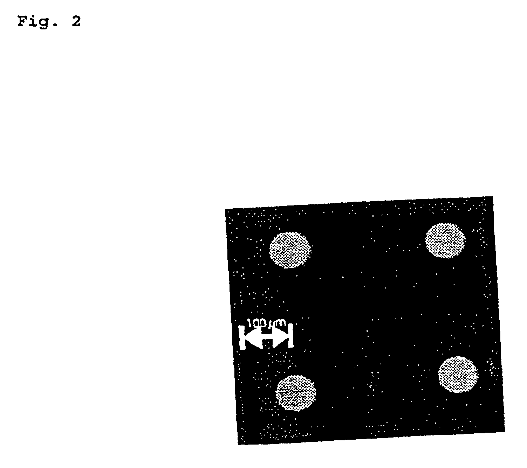

Ein Vergleich der in Figur 1 und Figur 2 gezeigten Testflächen demonstriert deutlich den durch den Waschschritt erhaltenen Vorteil, nämlich daß reproduzierbare, gleichmäßige Spotkonturen von Testflächen auf einem Reagenzfeld erhalten werden können.

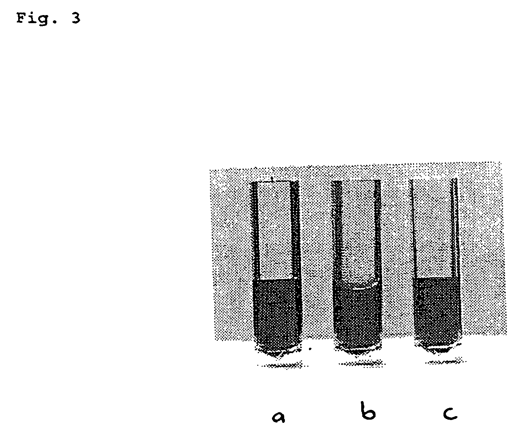

Befüllt man ein unbeschichtetes Enzymun-Teströhrchen mit Wasser, wie in Figur 3a gezeigt, so bildet sich eine völlig waagrechte Wasser-Luft-Grenzfläche aus. Dies bedeutet eine geringe Benetzung der Wand mit Wasser. Befüllt man hingegen ein mit Streptavidin vorbeschichtetes Enzymun-Teströhrchen mit Wasser, so ergibt sich eine stark gekrümmte Wasser-Luft-Grenzfläche, wie in Figur 3b gezeigt. Die Vorbeschichtung führt zu einer hohen Wandbenetzung. Befüllt man ein Teströhrchen, welches zunächst mit Streptavidin vorbeschichtet, dann intensiv mit einer wässrigen Flüssigkeit gewaschen und anschließend getrocknet wurde, so ergibt sich wiederum eine waagrechte Wasser-Luft-Grenzfläche, wie in Figur 3c gezeigt. Die Vorbeschichtung zeigt nach dem Waschschritt hydrophobe Eigenschaften, weshalb eine geringe Benetzung der Wand mit Wasser erfolgt.

Als fester, nicht poröser Träger kann erfindungsgemäß jedes feste, nicht poröse Material verwendet werden. Bevorzugt besteht der Träger aus Metall, Glas oder einem Kunststoff, insbesondere aus Polystyrol.

Die zweite Beschichtung wird bevorzugt in Form von räumlich begrenzten Flächen mit einem Durchmesser von 10 µm bis 10 mm aufgebracht. Da sich das erfindungsgemäße Verfahren insbesondere zur Herstellung von miniaturisierten Testformaten eignet, wird die zweite Beschichtung bevorzugt in Form von räumlich begrenzten Flächen mit einem Durchmesser von 10 µm bis 500 µm, besonders bevorzugt von 20 µm bis 200 µm aufgebracht. Das Aufbringen der zweiten Beschichtung kann mit bekannten Verfahren erfolgen. Zweckmäßigerweise wird die zweite Beschichtung in einer wässrigen Lösung in Form von Mikrotröpfchen mit einem Volumen zwischen 0,00001 bis 1 µl aufgebracht, z.B. mit Hilfe eines Flüssigkeitstrahlverfahrens. Es ist aber erfindungsgemäß auch möglich, größere Reaktionsgefäße, wie z.B. Mikrotiterplatten oder Küvetten zu beschichten.

Bevorzugt wird das Reagenzfeld des festen Trägers vollflächig vorbeschichtet, wonach auf diese vollflächige Vorbeschichtung die zweite Beschichtung in Form von räumlich begrenzten Testflächen aufgebracht wird. Es ist aber auch möglich, bereits die Vorbeschichtung in Form von Spots aufzubringen, was jedoch in einen höheren Aufwand resultiert, da die zweite Beschichtung dann genau auf die vorbeschichteten Flächen positioniert werden muß.

Die Reproduzierbarkeit des erfindungsgemäßen Verfahrens ermöglicht es, Testsysteme bereitzustellen, die mehrere gleiche oder verschiedene Testflächen, insbesondere in miniaturisierter Form enthalten, so daß sowohl die Menge des benötigten Analyten als auch die Analysezeiten deutlich verringert werden können. Es ist deshalb bevorzugt, mehrere räumlich begrenzte Flächen der zweiten Beschichtung auf einen Träger aufzubringen. Die Flächen können dabei alle die gleichen Rezeptormoleküle aufweisen. In diesem Fall kann eine Vielzahl von verschiedenen Proben gleichzeitig auf einem Testträger untersucht werden. Wenn mindestens zwei der Flächen jeweils unterschiedliche Rezeptormoleküle aufweisen, können in einer Probe mehrere Analyten parallel bestimmt werden. Bei solchen Systemen ist es besonders wichtig, räumlich scharf begrenzte Testflächen zu erzeugen und jegliches Verschmieren der Rezeptormoleküle zu vermeiden. Nur so kann sichergestellt werden, daß tatsächlich der gewünschte Analyt, der an eine spezifische Testfläche bindefähig ist, bestimmt wird und nicht eine Kontamination benachbarter Flächen mit Rezeptormolekülen anderer Testflächen zu falschen Resultaten führt.

Ein weiterer Gegenstand der Erfindung ist eine Festphase, die durch das zuvor beschriebene Verfahren erhältlich ist, umfassend eine Vorbeschichtung auf einen festen Träger und eine an die Vorbeschichtung in Form von mehreren, kreisförmigen räumlich begrenzten Flächen gebundene zweite Beschichtung, welche dadurch gekennzeichnet ist, daß die zweite Beschichtung an die Vorbeschichtung gebundene Rezeptormoleküle umfaßt und daß der Durchmesser der Flächen der zweiten Beschichtung 10 µm bis 10 mm beträgt und von Fläche zu Fläche weniger als 10%, bevorzugt um weniger als 5% und besonders bevorzugt um weniger als 2,5% variiert. Besonders bevorzugt wird diese Festphase in einem miniaturisierten Testsystem verwendet, wobei der Durchmesser der Testflächen 10 bis 500 µm, bevorzugt 20 bis 200 µm beträgt.

Weiterhin umfaßt die Erfindung die Verwendung einer solchen Festphase zum Nachweis eines oder mehrerer Analyten, insbesondere in Bindungsassays, wie etwa einem Immunoassay, einem Nukleinsäurehybridisierungsassay usw.

Ein weiterer Gegenstand der Erfindung ist ein Verfahren zur Verbesserung der Gleichmäßigkeit räumlich begrenzter Beschichtungsflächen auf einem festen, insbesondere nicht porösen Träger, umfassend die Schritte:
(a) Aufbringen einer Vorbeschichtung auf ein Reagenzfeld des festen Trägers,
(b) Waschen des vorbeschichteten Trägers mit einer wässrigen Flüssigkeit, die eine Konzentration an Puffersubstanzen von ≤ 250 mmol aufweist, und
(c) Aufbringen einer zweiten Beschichtung in Form von Mikrotröpfchen, die mit der Vorbeschichtung bindefähig sind, auf den vorbeschichteten Träger in Form von räumlich begrenzten Flächen auf dem Reagenzfeld.

Die Erfindung wird durch die folgenden Beispiele weiter erläutert:

### Beispiele

### Beispiel 1 Herstellung von Spots auf einer streptavidinbeschichteten Oberfläche

Die Testflächen wurden mit einer Beschichtungslösung beaufschlagt, die eine hochmolekulare Verbindung RSA-Streptavidin mit einer Partikelgröße von ca. 100 nm enthielt. Nach einer Reaktionszeit von 15 min-wurde die Beschichtungslösung abgesaugt und die Testflächen mit einer Lösung bestehend aus 0,05 % NaCl, 0,2% Saccharose und 0,05% RSA geblockt. Bei der in Fig. 1 gezeigten Testfläche wurde nach einer Inkubationszeit der Vorbeschichtung von 5 min nur trocken gesaugt, während bei den in Fig. 2 gezeigten Testflächen ein erfindungsgemäßer Waschschritt mit einem wäßrigen Medium durchgeführt wurde und anschließend trockengesaugt wurde.

### Beispiel 2

3,7 g Tris (Tris(hydroxymethyl)-aminomethan und 4,75 g MES (2-Morpholinoethansulfonsäure) werden in 5 l Wasser gelöst. Mit 5 ml dieses Waschpuffers wird ein vorbeschichtetes Reaktionsgefäß mit einem Nennvolumen von 50 µl überströmt. Anschließend wird das Reaktionsgefäß mit einer oder mehreren Absaugnadeln trockengesaugt. Durch Aufbringen einer zweiten Beschichtung auf die gewaschene und getrocknete Vorbeschichtung erhält man räumlich eng begrenzte Testflächen, wie sie beispielsweise in Figur 2 dargestellt sind.

### Beispiel 3

Auf einen festen Träger wird eine Streptavidinvorbeschichtung aufgebracht. Auf diese Beschichtung wird eine Beschichtungslösung mit monobiotinylierte Antikörper in Form kleiner Tröpfchen aufgegeben. Nachdem die Tröpfchen eingetrocknet sind, wird darauf eine Lösung gegeben, die 3 mg/ml Biotin in 50 mM K₂HPO₄ enthält. Nach einigen Sekunden wird die Biotinlösung entfernt und eine Lösung mit 1 % RSA (Rinderserumalbumin) und 2% Saccharose aufgebracht, welche nach wenigen Sekunden ebenfalls entfernt wird. Anschließend wird das System getrocknet. Auf diese Weise werden räumlich scharf begrenzte Festphasen erhalten, ohne Verschmierung von Rezeptormolekülen außerhalb der gewünschten Reaktionsfläche.

### Beispiel 4 Streptavidin-Vorbeschichtung

Zunächst wird ein biotinyliertes Makromolekül (z.B. thermisch aggregiertes RSA) in einer Konzentration von 100 µg/ml in PBS auf einen Polystyrolträger mit 50 µl Nennvolumen aufgebracht und 5 min inkubiert. Anschließend wird abgesaugt und eine Streptavidinlösung mit einer Konzentration von 100 µg/ml in PBS zugegeben. Nach 5 min Inkubation erfolgt ein Waschschritt mit anschließendem Trockensaugen.

Unterschiedliche, biotinylierte. DNA-Fangsonden (18-mer) werden in einer Konzentration von 1 µM in einen Puffer aus 5 mM Mes, 5 mM Tris, 1 % Saccharose und 0,5 mg/ml RSA gelöst. Anschließend werden Tröpfchen mit einem Volumen von ca. 150 µl in Form eines Arrays auf einen Polystyrolträger aufgebracht. Nach einer Inkubation für ca. 5 s und Trocknen für ca. 60 s wird eine Lösung bestehend aus 3 mg/ml Biotin in 50 mM Dikaliumphosphat zugegeben und für 2 s inkubiert. Anschließend wird abgesaugt und eine Blocklösung, bestehend aus 1% RSA und 2% Saccharose zugegeben, abgesaugt und getrocknet.

### Beispiel 5 Vorbeschichtung mit Anti-Maus-Antikörpern

PAK <Maus-F_{c*y*}> S IgG werden in einer Konzentration von 30 µg/ml in einem Puffer umfassen 40 mM Kaliumphosphat bei einem pH-Wert von 7,4 in 0,9% Natriumchlorid gelöst. Diese Lösung wird in ein Reaktionsgefäß mit 50 µl Nennvolumen aufgebracht. Nach 15 minütiger Inkubation und anschließendem Absaugen erfolgt die Zugabe einer Blocklösung bestehend aus 1 % RSA und 2% Saccharose. Nach 5 min Inkubation erfolgt erfindungsgemäß ein Waschschritt mit anschließendem Trockensaugen.

Ein Fusionsprotein (Maus-F_{c*y*} + CD28) wird in einem Puffer umfassend 5 mM Mes, 5 mM Tris, 1 % Saccharose und 0,5 mg/ml RSA auf 100 µg/ml gelöst. Es werden Tröpfchen mit einem Volumen von ca. 150 pl auf den zuvor beschriebenen, beschichteten Polystyrolträger aufgebracht und für ca. 5 s inkubiert. Nach Trocknen für ca. 60 s erfolgte die Zugabe einer Blocklösung bestehend aus 50 mM Kaliumphosphatpuffer, pH 7,4, 100 µg/ml Maus IgG, 1 mg/ml RSA. Nach Inkubation für 10 s wird abgesaugt und eine Blocklösung bestehend aus 1 % RSA, 2% Saccharose zugegeben, abgesaugt und getrocknet.

### Beispiel 6 Testflächen für Anti-Rubella-Antikörper

Ein biotinyliertes Makromolekül (z.B. thermisch aggregiertes RSA) wird in einer Konzentration von 100 µg/ml in PBS auf einen Polystyrolträger mit 50 µl Nennvolumen aufgebracht, 5 min inkubiert und abgesaugt. Anschließend wird eine Streptavidinlösung mit einer Konzentration von 100 µg/ml in PBS zugegeben. Nach 5 min Inkubation erfolgt erfindungsgemäß ein Waschschritt mit anschließendem Trockensaugen.

Ein biotinylierter Anti-Rubella-Antikörper wird auf eine Konzentration von 50 µg/ml in einen Puffer aus 5 mM Mes, 5 mM Tris, 1 % Saccharose und 0,5 mg/ml RSA gelöst. Tröpfchen mit einem Volumen von ca. 150 pl werden auf dem zuvor beschriebenen Träger aufgebracht. Es erfolgt eine Inkubation für ca. 5 s und ein Trocknen für ca. 60 s. Anschließend wird eine Lösung bestehend aus 3 mg/ml Biotin in 50 mM Dikaliumphosphat zugegeben, für 2 s inkubiert und abgesaugt. Dann wird eine Lösung, bestehend aus 0,1% Tween 20, 1 % RSA, 3 U/ml Virus-Lysat (Rubella-Virus) in PBS zugegeben, für 20 min Inkubiert und dann abgesaugt. Anschließend wird eine Blocklösung, bestehend aus 1% RSA und 2% Saccharose zugegeben, abgesaugt und getrocknet.

## Patentansprüche

1. Verfahren zum Aufbringen einer mehrlagigen Beschichtung auf einen festen, nicht porösen Träger, umfassend die Schritte:
(a) Aufbringen einer Vorbeschichtung auf ein Reagenzfeld des festen Trägers,
(b) Waschen des vorbeschichteten Trägers mit einer wässrigen Flüssigkeit, die eine Konzentration an Puffersubstanzen von ≤ 250 mmol aufweist, und
(c) Aufbringen einer zweiten Beschichtung in Form von Mikrotröpfchen, umfassend Rezeptormoleküle, die mit der Vorbeschichtung bindefähig sind, auf den vorbeschichteten Träger in Form von räumlich begrenzten Flächen auf dem Reagenzfeld.

2. Verfahren nach Anspruch 1,
**dadurch gekennzeichnet,**
**dass** die zweite Beschichtung in Form von räumlich begrenzten Flächen mit einem Durchmesser von 10 µm bis 10 mm aufgebracht wird.

3. Verfahren nach Anspruch einem der vorhergehenden Ansprüche,
**dadurch gekennzeichnet,**
**dass** der feste Träger mit einem ersten Partner eines spezifischen Bindepaares vorbeschichtet wird und das Rezeptormolekül den zweiten Partner umfasst.

4. Verfahren nach einem der vorhergehenden Ansprüche,
**dadurch gekennzeichnet,**
**dass** mehrere räumlich beegrenzte Flächen der zweiten Beschichtung auf einen Träger aufgebracht werden, wobei mindestens zwei der Flächen jeweils unterschiedliche Rezeptormoleküle aufweisen.

5. Festphase, erhältlich durch ein Verfahren nach einem der Ansprüche 1 bis 4, umfassend eine Vorbeschichtung auf einem festen, nichtporösen Träger und eine an die Vorbeschichtung in Form von mehreren, kreisförmigen räumlich begrenzten Flächen gebundene zweite Beschichtung,
**dadurch gekennzeichnet,**
**dass** die zweite Beschichtung an die Vorbeschichtung gebundene Rezeptormoleküle umfasst und dass der Durchmesser der Flächen der zweiten Beschichtung 10 µm bis 10 mm beträgt und von Fläche zu Fläche um weniger als 10 % variiert.

6. Verwendung einer Festphase nach Anspruch 5 zum Nachweis eines oder mehrerer Analyten.

7. Verfahren zur Verbesserung der Gleichmäßigkeit räumlich begrenzter Beschichtungsflächen auf einem festen, nicht-porösen Träger, umfassend die Schritte:
(a) Aufbringen einer Vorbeschichtung auf ein Reagenzfeld des festen Trägers,
(b) Waschen des vorbeschichteten Trägers mit einer wässrigen Flüssigkeit, die eine Konzentration an Puffersubstanzen von ≤ 250 mmol aufweist, und
(c) Aufbringen einer zweiten Beschichtung in Form von Mikrotröpfchen, wobei die zweite Beschichtung mit der Vorbeschichtung bindefähig ist, auf einen vorbeschichteten Träger in Form von räumlich begrenzten Flächen auf dem Reagenzfeld.

## Claims

1. Process for the application of a multilayer coating on a solid, non-porous support comprising the steps:
(a) application of a precoating to a reagent field of the solid support
(b) washing the precoated support with an aqueous liquid which has a concentration of buffer substances of ≤ 250 mmol, and
(c) application of a second coating in the form of microdroplets comprising receptor molecules which can bind to the precoating on the precoated support in the form of spatially defined zones on the reagent field.

2. Process as claimed in claim 1,
**characterized in that**
the second coating is applied in the form of spatially defined zones with a diameter of 10 µm to 10 mm.

3. Process as claimed in one of the previous claims,
**characterized in that**
the solid support is precoated with a first partner of a specific binding pair and the receptor molecule comprises the second partner.

4. Process as claimed in one of the previous claims,
**characterized in that**
several spatially defined zones of the second coating are applied to a support where at least two of the zones each have different receptor molecules.

5. Solid phase obtainable by a process as claimed in claims 1 to 4, comprising a precoating on a solid, non-porous support and a second coating bound to the precoating in the form of several, circular spatially defined zones,
**characterized in that**
the second coating comprises receptor molecules bound to the precoating and the diameter of the zones of the second coating is 10 µm to 10 mm and varies by less than 10 % from zone to zone.

6. Use of a solid phase as claimed in claim 5 for the detection of one or more analytes.

7. Process for improving the uniformity of spatially defined coating zones on a solid, non-porous support comprising the steps
(a) application of a precoating to a reagent field of the solid support,
(b) washing the precoated support with an aqueous liquid which has a concentration of buffer substances of ≤ 250 mmol, and
(c) application of a second coating in the form of microdroplets which can bind to the precoating on the precoated support in the form of spatially defined zones on the reagent field.

## Revendications

1. Procédé de dépôt d'un revêtement multicouches sur un support solide non poreux, comprenant les étapes de :
(a) dépôt d'un pré-revêtement sur un champ réactif du support solide,
(b) lavage du support pré-revêtu avec un liquide aqueux, qui présente une concentration en substances tampons ≤ 250 mmol, et
(c) dépôt d'un deuxième revêtement sous la forme de microgouttelettes, comprenant des molécules réceptrices, qui sont capables de se lier au pré-revêtement, sur le support prérevêtu sous la forme de surfaces limitées dans l'espace sur le champ réactif.

2. Procédé selon la revendication 1, **caractérisé en ce que** le deuxième revêtement est déposé sous la forme de surfaces limitées dans l'espace avec un diamètre de 10 µm à 10 mm.

3. Procédé selon l'une des revendications précédentes, **caractérisé en ce que** le support solide est pré-revêtu avec un premier partenaire d'un couple de liaison spécifique et que la molécule réceptrice comprend le deuxième partenaire.

4. Procédé selon l'une des revendications précédentes, **caractérisé en ce que** plusieurs surfaces limitées dans l'espace du deuxième revêtement sont déposées sur un support, au moins deux des surfaces présentant respectivement différentes molécules réceptrices.

5. Phase solide susceptible d'être obtenue par un procédé selon l'une des revendications 1 à 4, comprenant un pré-revêtement sur un support solide non poreux et un deuxième revêtement lié au pré-revêtement sous la forme de plusieurs surfaces circulaires limitées dans l'espace, **caractérisé en ce que** le deuxième revêtement comprend des molécules réceptrices liées au pré-revêtement et **en ce que** le diamètre des surfaces du deuxième revêtement est 10 µm à 10 mm et qu'il varie de surface en surface de moins de 10 %.

6. Utilisation d'une phase solide selon la revendication 5 pour la détection d'un ou de plusieurs analytes.

7. Procédé pour améliorer l'uniformité de surfaces de revêtement limitées dans l'espace sur un support solide non poreux, comprenant les étapes de :
(a) dépôt d'un pré-revêtement sur un champ réactif du support solide,
(b) lavage du support pré-revêtu avec un liquide aqueux, qui présente une concentration en substances tampons ≤ 250 mmol, et
(c) dépôt d'un deuxième revêtement sous la forme de microgouttelettes, le deuxième revêtement étant capable de se lier au pré-revêtement, sur le support prérevêtu sous la forme de surfaces limitées dans l'espace sur le champ réactif.
